(19) Europäisches Patentamt
European Patent Office
Office européen des brevets

(11) EP 1 195 377 A1

(12) EUROPEAN PATENT APPLICATION
published in accordance with Art. 158(3) EPC

(43) Date of publication:
**10.04.2002 Bulletin 2002/15**

(21) Application number: **00944359.9**

(22) Date of filing: **07.07.2000**

(51) Int Cl.[7]: **C07D 407/12**, A61K 31/375, A61K 7/00, A61K 7/42, A61P 29/00

(86) International application number:
**PCT/JP00/04575**

(87) International publication number:
**WO 01/04114 (18.01.2001 Gazette 2001/03)**

(84) Designated Contracting States:
**AT BE CH CY DE DK ES FI FR GB GR IE IT LI LU MC NL PT SE**

(30) Priority: **08.07.1999 JP 19441399**

(71) Applicant: **Senju Pharmaceutical Co., Ltd.**
**Osaka-shi, Osaka 541-0046 (JP)**

(72) Inventors:
- **OGATA, Kazumi**
  **Toyonaka-shi, Osaka 565-0085 (JP)**
- **SAITO, Noriko**
  **Minoo-shi, Osaka 562-0046 (JP)**
- **OGINO, Shinya**
  **Itami-shi, Hyogo 664-0898 (JP)**

(74) Representative: **Burford, Anthony Frederick et al**
**W.H. Beck, Greener & Co.**
**7 Stone Buildings**
**Lincoln's Inn**
**London WC2A 3SZ (GB)**

# (54) DIESTERS OF MALEIC OR FUMARIC ACID

(57) Diesters of maleic or fumaric acid as represented by general formula (1) or pharmacologically acceptable salts thereof, which are useful as cosmetic ingredients, antioxidants, radical scavengers, or anti-inflammatory agents wherein $R_1$ and $R_2$ are each independently hydrogen or methyl.

EP 1 195 377 A1

## Description

Technical Field

The present invention relates to novel and useful diester compounds of maleic acid (or fumaric acid) and to their production process and uses. In more particular, the present invention relates to diester compounds of maleic acid (or fumaric acid) formed with tocopherols and ascorbic acid and to their production process and uses.

Background of the Invention

Vitamin C (ascorbic acid), which is an anti-scurvy agent, acts to suppress deposition of melanin pigments causing stains or blotches, freckles, etc. and is recently said to exhibit even anti-cancer activity. On the other hand, vitamin E (α-, β-, γ-and δ-tocopherols) possesses anti-oxidation activity and in recent years, is suggested to be effective against cataract. In this connection , attention should be drawn to the distinguishing feature that Vitamin E itself is insoluble in water, whereas ascorbic acid is soluble in water. As a derivative produced by combining vitamins C and E via a certain type of a compound, there have heretofore been known water-soluble diester compounds of phosphoric acid formed with vitamins E and C which were invented by one of the present inventors (JP-B-2-44478 and JP-B-5-23274). Also, tocopherol/tocotriphenol-L-ascorbic acid-6-dicarboxylic acid diesters were reported as a compound acting to make such contradictory solubilities of vitamins C and E compatible (JP-A-62-187470). Furthermore, α-tocopherolglyceryl ascorbate is known as a derivative of vitamins C and E (JP-A-5-331166).

Under these circumstances, the present inventors conducted intensive research on novel derivatives of vitamins C and E, and as a result, succeeded in synthesizing diesters of maleic acid (or fumaric acid) according to the present invention, leading moreover to the finding that the diester compounds exhibit excellent antioxidant, radical scavenging and anti-inflammatory activities. The finding has culminated in completion of the present invention.

Disclosure of the Invention

The present invention is intended to provide novel derivatives of vitamins C and E, or novel compounds having tocopherol and ascorbic acid bonded by ester linkages via maleic acid (or fumaric acid). Furthermore, the object of the present invention is to provide cosmetics, antioxidants, radical scavengers and anti-inflammatory agents which contain such novel compounds.

Namely, the present invention relates to:

(1) Diester compounds of maleic acid (or fumaric acid) (hereinafter referred to as " Compounds" ) represented by the following formula or their pharmacologically acceptable salts:

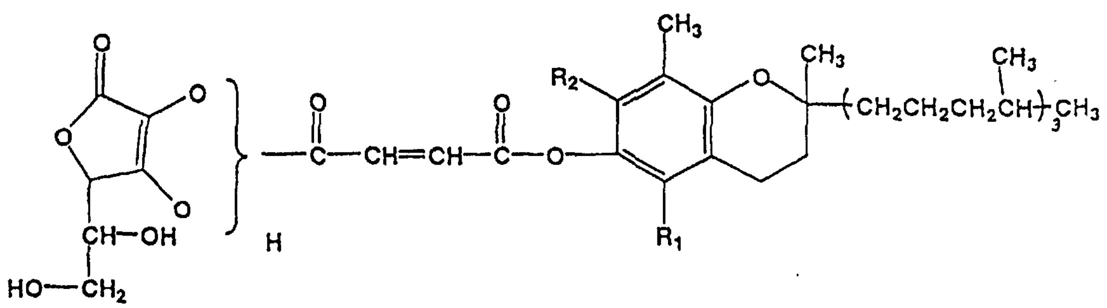

(wherein each of $R_1$ and $R_2$ is the same as, or different from, the other and designates a hydrogen atom or methyl group);

(2) The diester compounds or their pharmacologically acceptable salts as described above under the item (1), which are represented by α-tocopheryl L-ascorbic acid-2-O-maleate (or fumarate);

(3) The diester compounds or their pharmacologically acceptable salts as described above under the item (1), which are represented by α-tocopheryl L-ascorbic acid-3-O-maleate (or fumarate) ;

(4) A process for producing the diester compounds of maleic acid (fumaric acid) or their pharmacologically acceptable salts as described above under the item (1), characterized in that said process comprises esterifying monotocopheryl maleate (or fumarate) and ascorbic acid having its hydroxyl groups protected at the 5 and 6 positions in a highly polar organic solvent in the presence of a base in accordance with the mixed acid anhydride method,

followed by removal of the protective groups with an acid;

(5) Cosmetics which comprise the diester compounds or their pharmacologically acceptable salts as described above under the items (1) to (3);

(6) Antioxidants or radical scavengers which comprise the diester compounds or their pharmacologically acceptable salts as described above under the items (1) to (3);

(7) Pharmaceuticals which comprise the diester compounds or their pharmacologically acceptable salts as described above under the items (1) to (3);

(8) Anti-inflammatory agents which comprise the diester compounds or their pharmacologically acceptable salts as described above under the items (1) to (3);

(9) A method for absorbing ultraviolet radiation or suppressing deposition of melanin pigment which comprises applying to the skin in need thereof the diester compounds of maleic acid (or fumaric acid) or their pharmacologically acceptable salts represented by the formula described above under the item (1) ;

(10) A method as described above under the item (9), wherein the diester compounds or pharmacologically acceptable salts are represented by α- tocopheryl L-ascorbic acid-2-O-maleate (or fumarate);

(11) The method as described above under the item (9), wherein the above-described diester compounds or pharmacologically acceptable salts are represented by α-tocopheryl L-ascorbic acid-3-O-maleate (or fumarate);

(12) A method for suppressing oxidation or scavenging radicals which comprises administering to a subject in need thereof an effective amount of the diester compounds of maleic acid (or fumaric acid) or their pharmacologically acceptable salts represented by the formula as described above under the item (1);

(13) The method as described above under the item (12), wherein the above-described diester compounds or their pharmacologically acceptable salts are represented by α-tocopheryl L-ascorbic acid-2-O-maleate (or fumarate);

(14) The method as described above under the item (12), wherein the above-described diester compounds or their pharmacologically acceptable salts are represented by α-tocopheryl L-ascorbic acid-3-O-maleate (or fumarate);

(15) A method for preventing or treating inflammations which comprises administering to a subject in need thereof an effective amount of the diester compounds of maleic acid (or fumaric acid) or their pharmacologically acceptable salts represented by the formula as described above under the item (1);

(16) The method as described above under the item (15), wherein the above-described diester compounds or their pharmacologically acceptable salts are represented by α-tocopheryl L-ascorbic acid-2-O-maleate (or fumarate);

(17) The method as described above under the item (15), wherein the above-described diester compounds or their pharmacologically acceptable salts are represented by α-tocopherol L-ascorbic acid-3-O-maleate (or fumarate);

(18) Uses in the manufacture of cosmetics of the diester compounds of maleic acid (or fumaric acid) or their pharmacologically acceptable salts represented by the formula as described above under the item (1) ;

(19) The uses as described above under the item (18), wherein the above-described diester compounds or their pharmacologically acceptable salts are represented by α-tocopheryl L-ascorbic acid-2-O-maleate (or fumarate);

(20) The uses as described above under the item (18), wherein the above-described diester compounds or their pharmacologically acceptable salts are represented by α-tocopheryl L-ascorbic acid-3-O-maleate (or fumarate);

(21) Uses in the manufacture of antioxidants or radical scavengers of the diester compounds of maleic acid (or fumaric acid) or their pharmacologically acceptable salts represented by the formula as described above under the item (1);

(22) The uses as described above under the item (21), wherein the above-described diester compounds or their pharmacologically acceptable salts are represented by α-tocopheryl L-ascorbic acid-2-O-maleate (or fumarate);

(23) The uses as described above under the item (21), wherein the diester compounds or their pharmacologically acceptable salts are represented by α- tocopheryl L-ascorbic acid-3-O-maleate (or fumarate);

(24) Uses in the manufacture of pharmaceuticals of the diester compounds of maleic acid (or fumaric acid) or their pharmacologically acceptable salts represented by the formula as described above under the item (1) ;

(25) The uses as described above under the item (24), wherein the above-described diester compounds or their pharmacologically acceptable salts are represented by α-tocopheryl L-ascorbic acid-2-O-maleate (or fumarate);

(26) The uses as described above under the item (24), wherein the above-described diester compounds or their pharmacologically acceptable salts are represented by α-tocopheryl L-ascorbic acid-3-O-maleate (or fumarate);

(27) Uses in the manufacture of anti-inflammatory agents of the diester compounds of maleic acid (or fumaric acid) or their pharmacologically acceptable salts represented by the formula as described above under the item (1);

(28) The uses as described above under the item (27), wherein the above-described diester compounds or their pharmacologically acceptable salts are represented by α-tocopheryl L-ascorbic acid-2-O-maleate (or fumarate); and

(29) The uses as described above under the item (27), wherein the above-described diester compounds or their pharmacologically acceptable salts are represented by α-tocopheryl L-ascorbic acid-3-O-maleate (or fumarate).

Brief Description of the Drawings

Fig. 1 is an infrared absorption spectrum (IR) of the compound as synthesized in Example 1.

Detailed Description of the Invention

As described in the above, the Compounds have the structure consisting of ascorbic acid and tocopherol bonded by ester linkages via maleic acid (or fumaric acid). The diester compound of maleic acid (cis-isomer) and the diester compound of fumaric acid (trans-isomer) according to the present invention are considered to be formed in a fixed proportion depending upon the reaction conditions, etc. Both of the diester compounds can suitably be used for the purposes of the present invention.

The Compounds, in the forms of either free acids or their pharmacologically acceptable salts, can suitably be utilized for the purposes of the present invention. Their pharmacologically acceptable salts are exemplified by their alkali metal salts, such as their sodium and potassium salts, and their alkaline earth metal salts, such as their calcium and magnesium salts. Any of miscellaneous salts of the Compounds can properly be used, only if they are pharmacologically acceptable.

As vitamin E which is one constituent of the Compounds, any of α-, β-, γ- and δ-tocopherols can suitably be used. As mentioned previously, vitamin E is an antioxidant, and is recently suggested to be effective against cataract.

As mentioned above, ascorbic acid, which is another constituent of the Compounds, is an anti-scurvy agent and also acts to suppress deposition of melanin pigment causing stains or blotches, freckles, etc., while furthermore in recent years, ascorbic acid is said to exhibit anti-cancer activity.

The Compounds have the structure in which ascorbic acid has either of hydroxy groups esterified at the 2 or 3 position.

The Compounds can properly be synthesized, for example, by the below-described method or some methods similar to the same: monotocopheryl maleate (or fumarate) and ascorbic acid having the hydroxyl groups protected at the 5 and 6 positions can be esterified in a highly polar solvent in the presence of a base, such as alkali carbonate compounds and trimethylamine in accordance with the mixed acid anhydride method, followed by removal of the protective groups to give the Compound having ascorbic acid esterified mainly at the 2 position. As a highly polar organic usable in this reaction, there may be mentioned, for example, dimethylsulfoxide (DMSO), dimethylformamide (DMF) and acetonitrile. Examples of the alkali carbonate compounds include sodium carbonate and potassium carbonate.

In connection to the above, it is to be mentioned that when tetrahydrofuran (THF) is used as a solvent with pyridine being utilized as a base, the diester compound having ascorbic acid bonded at the 3 position is mostly yielded in the form of an oily product.

The Compounds obtained in the above-described manner may be converted into their pharmacologically acceptable salts by conventionally known methods. The conversion into such salts may be effected after isolation from the reaction solution or directly without such isolation.

Monotocopheryl maleate (or fumarate), which is a starting compound for the Compounds, can be synthesized by the procedure described in PCT/JP98/05765 or some procedures similar to the same. Namely, tocopherol and maleic anhydride can be reacted by heating for 1 to 3 hours in a solvent, such as acetone, acetonitrile or tetrahydrofuran (THF), in the presence of an alkali carbonate (e.g., sodium carbonate, potassium carbonate, etc.) or an alkali acetate (e.g., sodium acetate, potassium acetate, etc.) to give monotocopheryl maleate (or fumarate).

Ascorbic acid having protective groups for the hydroxyl groups at the 5 and 6 positions, which is another starting compound for the Compounds, can be synthesized by conventionally known methods, for example, the methods described in the above-mentioned JP-B-2-44478 and JP-B-5-23274 or some methods similar to the same. The protective groups for the hydroxyl groups at the 5 and 6 positions of ascorbic acid may include, for example, acyl groups, such as isopropylidene, benzylidene and acetyl groups, with isopropylidene group being commonly used. Such protective groups can be removed easily by acidifying the reaction solution. On the occasion of such acidification, there can be utilized inorganic acids, such as hydrochloric acid, phosphoric acid and sulfuric acid, and organic acids, such as acetic acid and citric acid.

The Compounds are novel compounds which have not been described in literature, and possess the advantage that they are water-soluble and oil-soluble. Consequently, the Compounds can advantageously be used in the forms of cosmetics, injections and ophthalmic solutions or eye drops. The diester compounds having ascorbic acid linked at the 3 position, which is less water-soluble than the diester compounds having ascorbic acid linked at the 2 position, can find application in lotions or ointments, etc.

The Compounds dissociate into ascorbic acid, tocopherol and maleic acid (or fumaric acid) in vivo, and is therefore useful as a prodrug for ascorbic acid and tocopherol. In addition, the Compounds with their oil-solubility as

mentioned previously can offer the advantage that they exceed in biopermeability. Since the Compounds can be expected to produce sufficiently the effect of ascorbic acid in spite of the serious drawback of inferior skin permeability owing to its inherent water solubility, accordingly, they can advantageously be used as a cosmetic, etc.

The Compounds possess excellent anti-inflammatory, antioxidant and radical scavenging activities, as may be evident from the below-described examples.

The Compounds, with their excellent anti-inflammatory, antioxidant and radical scavenging activities, can properly be added to cosmetics, such as creams, lotions and skin lotions, for the purposes of ultraviolet radiation absorption (e.g., repression of rubedo caused by ultraviolet irradiation, prevention of sun tanning, etc.) and skin beauty and whitening (e.g., prevention of deposition of melanin pigment which causes stains or blotches and freckles, etc.) as well as for the purpose of stabilization (anti-oxidation) of ingredients for cosmetics.

In formulating the Compounds into cosmetics, there can suitably be added the ingredients which are conventionally utilized in cosmetics. Such ingredients include, for example, nicotinic acids, such as nicotinic acid, nicotinamide and benzyl nicotinate, vitamin As, such as retinol, retinolyl acetate and vitamin A oil, vitamin $B_2$s, such as riboflavin, riboflavin acetate and flavinadenine dinucleotide, vitamin $B_6$s, such as pyridoxine hydrochloride and pyridoxine dioctanoate, vitamin Cs, such as L-ascorbic acid, sodium L-ascorbic acid-2-sulfate and L-ascorbyl dipalmitate, pantothenic acids, such as calcium pantothenate, pantothenyl ethyl ether, D-pantothenyl alcohol and acetylpantothenyl ethyl ether, vitamin Ds, such as cholecalcipherol and ergocalcipherol, vitamin Es, such as α-tocopherol, tocopherol acetate, DL-α-tocopheryl nicotinate, DL-α-tocopheryl succinate, other vitamins; amino acids, such as glycine, alanine, phenylalanine, valine, leucine, isoleucine, serine, threonine, asparagine, aspartic acid, aspartates, glutamine, glutamic acid, glutamates, lysine, methionine, cysteine, cystine, arginine, histidine, tryptophan, proline and hydroxyproline, N-acylated acidic amino acid salts, such as sodium N-coconut oil fatty acid-L-glutamate and diethyl N-palmitoyl-L-aspartate, acylated neutral amino acid salts, such as sodium lauroylmethyl-β-alanate and coconut oil fatty acid succinotriethanolamide, pyrrolidonecarboxylic acid and its salts, amino acid derivatives, such as polyoxyethylenated hardened castor oil monopyroglutamate monostearate diester, coconut oil fatty acid-L-arginine ethyl ester-DL-pyrrolidonecarboxylate, oils, such as rice bran oil, peanut oil, palm oil, beef tallow, avocado oil, hohoba oil, lanolin, liquid paraffin, squalane, carnauba wax, isostearyl alcohol, isostearyl palmitate and glyceryl tri-2-ethylhexanoate, polyhydric alcohols, such as glycerol, sorbitol, mannitol and 1,3-butylene glycol, polyhydric alcohol ether, such as polyethylene glycol, mucic polysaccharides, such as collagen, sodium hyaluronate, sodium chondroitin sulfate and sodium dextran sulfate, antioxidants, such as p-hydroxyanisole and sodium erythorbate, cellulose derivatives, such as carboxyvinyl polymer, carboxymethylcellulose and hydroxypropyl methylcellulose, surfactants, such as sodium stearyl sulfate, diethanolamine cetylsulfate, cetyl trimethylammonium saccharine, isostearyl polyethylene glycol, diglyceryl diisostearate and phospholipids, preservatives, such as ethylparaben, propylparaben and butylparaben, anti-inflammatory agents, such as hinokitiol, salicylic acid derivatives, glycyrrhetic acid derivatives, allantoin and zinc oxide, miscellaneous pH regulating agents, buffers, perfumes and coloring agents.

In cases where the Compounds are used in cosmetics, they are normally formulated in a proportion of about 0.001 to 5 (w/w)%, preferably about 0.005 to 2 (w/w)%, although such proportion may be varied depending upon the type of the Compounds, the type of a cosmetic to be processed through formulation and the purpose of formulation.

The Compounds exhibit anti-inflammatory activity as described in the above, and the particular inflammatory disorders to be treated with the Compounds may include, for example, hemorrhoids, rheumatoid arthritis, rheumatism deformans, spondylitis deformans, arthritis deformans, back pain, onset of gout, acute otitis media, cystitis, prostatitis, odontalgia, conjunctivitis, keratitis, iridocyclitis, uveitis and sinusitis.

In cases where the Compounds are used as an anti-inflammatory agent, one or more than two types of the Compounds are contained in appropriate combination in accordance with the purpose and need.

The Compounds may suitably be usable orally or parenterally as an anti-inflammatory agent, and can be processed into any dosage forms, such as solid pharmaceutical preparations being exemplified by tablets, granules, powders, capsules, etc. or liquid pharmaceutical preparations being exemplified by injections, ophthalmic solutions, etc., by the conventionally known processes. A variety of ordinarily used additives or pharmaceutic aids, such as excipients, binders, thickeners, dispersing agents, reabsorption promoters, buffers, surfactants, solubilizing agents, preservatives, emulsifying agents, tonicity agents, stabilizing agents and pH regulating agents, may suitably used in such dosage forms.

In using the Compounds as an anti-inflammatory agent, the dose may be varied depending upon the type of the Compounds to be used, the body weight and age of a patient, the sort and conditions of a disorder to be treated and the method of administration, and they may desirably be administered to adult patients at doses ranging from about 1 mg to about 30 mg once a day in the case of injections, and at doses ranging from about 1 mg to about 100 mg each time and several times a day in the case of pharmaceuticals for internal use. In the case of ophthalmic solutions, an ophthalmic solution of a concentration varying from about 0.01 to 5 (w/v)% is preferably instilled or applied topically to the eye in several drops each time and several times a day.

The anti-inflammatory agents comprising the Compounds may be incorporated with any other anti-inflammatory agents or different types of active agents, unless they are contradictory to the purposes of the present invention.

The Best Mode for carrying Out the Invention

Given below are the examples and preparation-processing examples to illustrate the present invention in more detail, but the scope of the present invention is not understood to be limited to them.

Example 1: α-Tocopheryl L-ascorbic acid-2-O-maleate (or fumarate)

A 60 ml volume of acetone is added to 4.3 g (0.01 mole) of α-tocopherol, 3.0 g (0.03 mole) of maleic anhydride and 1.5 g of sodium acetate, followed by heating for 90 min under stirring, and acetone is distilled off. The resultant residue is treated with water, and the solution is acidified with hydrochloric acid, followed by extraction with ethyl acetate. The extract is washed with water and then freed of ethyl acetate by distillation to give 5.3 g of a residual oily product (which crystallizes when left on standing).

Monotocopheryl maleate (or fumarate) obtained by the above procedure is dissolved in 40 ml of chloroform, and the solution is admixed with 1.2 g of triethylamine, followed by cooling at about -5°C. 1.3 g of ethyl chlorocarbonate is gradually added dropwise to the reaction solution under stirring, to which a solution prepared by dissolving 2.5g of 5,6-isopropylideneascorbic acid in 40 ml of DMF and adding for neutralization 1.4 g of anhydrous potassium carbonate is added once 15 min. later. The reaction mixture is stirred for 30 min., then cooled down to room temperature and further stirred for 1 hour, and is admixed with 50 ml of water and acidified with hydrochloric acid. After chloroform is distilled off, the reaction product is extracted with ethyl acetate and the extract is freed of ethyl acetate by distillation. The resultant residual oily product is dissolved in 60 ml of ethanol, and the solution is admixed with 15 ml of 1N-hydrochloric acid, followed by heating at 60°C for 15 min. under stirring. After the solvent is distilled off, the resultant residue is extracted with ethyl acetate, and the extract is washed and then freed of ethyl acetate by distillation to give a residual solid. The residual solid is treated with n-hexane, and the resultant white crystals are recovered by filtration and recrystallized from ethyl acetate/ethanol/n-hexane to give 3.6 g of the objective compound. m.p. 86-88°C. TLC, Rf = 0.67 (n-butanol:acetic acid:water = 4:1:1) . Its IR is shown in Fig. 1.

| Elemental analysis, for $C_{39}H_{58}O_{10} \cdot H_2O$ | | |
|---|---|---|
| Calcd.(%) | C, 66.45; | H, 8.58 |
| Found (%) | C, 66.35; | H, 8.57 |

Example 2: α-Tocopheryl L-ascorbic acid-2-O-maleate (or fumarate) (Alternative method)

By following the same procedure as described in Example 1 except that 4.3 g (0.01 mole) of dl-α-tocopherol is used, there is obtained 5.3 g of monotocopheryl maleate (or fumarate).

The compound is dissolved in 30 ml of tetrahydrofuran, to which 2.0 g of tributylamine is added, followed by cooling to about -5°C. 1.2 g of ethyl chlorocarbonate is gradually added dropwise to the solution under stirring, to which a solution prepared by dissolving 2.5 g of 5,6-isopropylideneascorbic acid and 1.5 g of triethylamine in 40 ml of acetonitrile is added once 20 min. later, followed by stirring for 30 min. and then at 5°C for 1 hour. The reaction solution is acidified with 20 ml of 2N-hydrochloric acid added, and freed of the solvent by distillation under reduced pressure. The residue is extracted with diisopropyl ether, and the extract is washed with water and freed of the solvent by distillation. The resultant residual oily product is dissolved in 60 ml of ethanol, and the solution is admixed with 15 ml of 1N-hydrochloric acid and heated at 60°C for 40 min. under stirring to remove the protective groups. By following the same procedure as described in Example 1 hereafter, there are obtained crude crystals, which are recrystallized from ethyl acetate ethanol/diisopropyl ether to give 3.1 g of the objective compound.

Example 3: α-Tocopheryl L-ascorbic acid-3-O-maleate (fumarate)

While replacing 40 ml of THF for DMF and 2 ml of pyridine for anhydrous potassium carbonate, the reaction treatment is carried out in the same manner as described in Example 1 to give 6.5 g of an oily product, which is then purified by chromatography on a silica gel column with use of n-hexane/chloroform as an eluent to yield 3.0 g of the objective compound in the form of a yellowish oily product. TLC, Rf = 0.85 (chloroform:methanol=4:1).

Example 4: The radical-scavenging capacity of the Compound

The Compound is tested for the radical scavenging capacity against the stabilized radical of 1,1-diphenyl-2-picrylhydro radicals (DPPH).

[Test substance]

The compound of Example 1 is dissolved in ethanol to the concentrations ranging from $10^{-2}$ to $10^{-5}$M.

[Test method]

The test is carried out in accordance with the procedure of Blois, M.S., Nature; 181, 1199 (1985).
Added to 300 µl of a sample solution is 2.7 ml of a 0.011 mM ethanol solution of DPPH or a stabilized radical, and the solution is stirred and left on standing for 20 min., followed by measurement of absorbance at a wavelength of 517 nm. The radical scavenging rate of the sample solution is determined by the following equation:

Radical scavenging rate (%) = (absorbance of control -

absorbance of the sample solution x absorbance of control)

x 100

[Test results]

The results are tabulated in Table 1.

Table 1:

| The radical scavenging capacity of the Compound | | |
|---|---|---|
| Test substance | Final concn. (µM) | Radical scavenging capacity |
| The compound of Example 1 | 1000 | 61.4 |
| | 300 | 29.5 |
| | 100 | 10.8 |
| | 10 | 5.2 |

The Compound is found to exhibit DPPH radical scavenging activity, with $IC_{50}$ value being at about 560 µM.

Example 5: Effect of the Compound against the inflammation model of mouse auricula

[Test substance]

The compound of Example 1 is dissolved in ethanol to the concentration of 0.5%.

[Test method]

6-Weeks old male mice of ddy strain as procured from SLC CO. are subjected to the test.
In order to cause inflammation, a solution (100 µg/mL) of phorbol 12-myristate 13-acetate (PMA) in acetone is applied in 10 µl portions (20 µl in total per mouse) onto the inside and outside of right-hand auricula of each mouse in such a manner that the solution may extend over the whole auricula. 24 Hours after causing inflammation, the thickness of right-hand auricula of each mouse is measured with use of a dial thickness gauge, and the edematization rate is calculated based on the thickness before causing inflammation.
The test substance is applied in 10 µl portions (20 µl in total per mouse) onto the inside and outside of right-hand auricula of each mouse.
The results are tabulated in Table 2.

Table 2:

| Effect of the Compound against the mouse auriculs inflammation | | |
|---|---|---|
| Test substance | Edematization rate (%) | Inhibition rate (%) |
| Control (methanol) | 166.1 ± 19.0 | - |
| Compound of Example 1 | 26.9 ± 6.2* | 83.8 |

Remarks: The values designate mean ± S.E. (n=8).
Significance against control (methanol); *; $p<0.001$

As is evident from Table 2, the Compound, which shows auricula edematization inhibition rate of 83.3%, can inhibit edematization significantly against the control group (methanol).

The results demonstrate that the Compound is useful as an anti-inflammatory agent.

| Preparation-Processing Example 1: Beauty lotion | |
|---|---|
| Compound of Example 1 | 0.2 g |
| Polyvinylpyrrolidone | 1.0 g |
| Polyoxyethylenated hardened castor oil (HCO-60) | 1.0 g |
| Ethanol | 15 ml |
| Triethanolamine | Appropriate volume |
| Methyl p-oxybenzoate | 0.1 g |
| Propyl p-oxybenzoate | 0.05 g |
| Sterilized purified water | Made up to the total of 100 ml pH3.5 |

The above-described ingredients are mixed by the conventionally known process to give a beauty lotion.

| Preparation-Processing Example 2: Tablet for internal use | |
|---|---|
| Compound of Example 1 | 30 mg |
| Lactose | 80 mg |
| Potato starch | 17 mg |
| Polyethylene glycol 6000 | 3 mg |

The above-described ingredients as a starting material for one tablet are compressed into a tablet by the conventionally known process.

| Example 3: Cosmetic | |
|---|---|
| Compound of Example 1 | 0.5 g |
| Olive oil | Made up to the total of 100 ml |

The above-described ingredients are dissolved to give an oily cosmetic.

Industrial Applicability

The Compounds are novel compounds which have not been described in literature, and can offer the advantage that they are water-soluble and oil-soluble, as has been described above. Consequently, the Compounds especially can advantageously be used in the forms of cosmetics, injections and ophthalmic solutions or eye drops.

The Compounds can be expected to dissociate into ascorbic acid, tocopherol and maleic acid (or fumaric acid) in vivo, and is therefore useful as a prodrug for ascorbic acid and tocopherol. In addition, the Compounds with their oil-solubility as mentioned previously can have the advantage that they exceed in biopermeability. Since the Compounds can be expected to produce sufficiently the effect of ascorbic acid in spite of the serious drawback of inferior skin permeability brought about by its inherent water solubility, accordingly, they can advantageously be used as a cosmetic, etc.

## Claims

1. A diester compound of maleic acid (or fumaric acid) represented by the following formula or its pharmacologically acceptable salt:

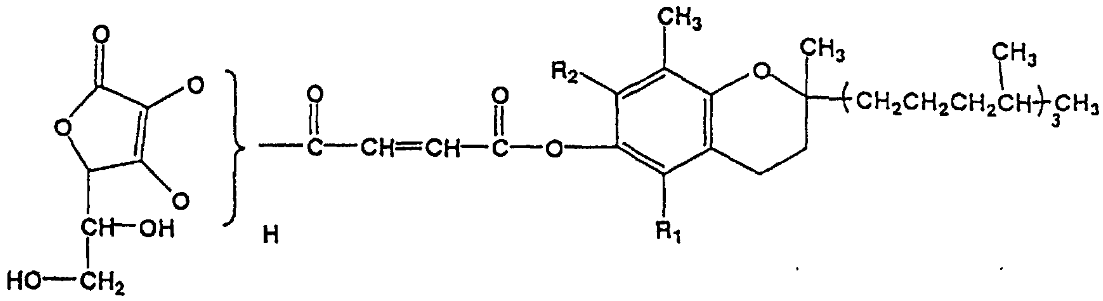

(wherein each of $R_1$ and $R_2$ is the same as, or different from, the other and designates a hydrogen atom or methyl group).

2. A diester compound or its pharmacologically acceptable salt as claimed in claim 1, which is represented by α-tocopheryl L-ascorbic acid-2-O-maleate (or fumarate).

3. A diester compound or its pharmacologically acceptable salt as claimed in claim 1, which is represented by α-tocopheryl L-ascorbic acid-3-O-maleate (or fumarate).

4. A process for producing a diester compound of maleic acid (or fumaric acid) or its pharmacologically acceptable salt as claimed in claim 1, **characterized in that** said process comprises esterifying monotocopheryl maleate and ascorbic acid having its hydroxyl groups protected at the 5 and 6 positions in a highly polar organic solvent in the presence of a base in accordance with the mixed acid anhydride method, followed by removal of the protective groups with an acid.

5. A cosmetic which comprises a diester compound or its pharmacologically acceptable salt as claimed in claims 1 to 3.

6. An antioxidant or radical scavenger which comprises a diester compound or its pharmacologically acceptable salt as claimed in claims 1 to 3.

7. A pharmaceutical which comprises a diester compound or its pharmacologically acceptable salt as claimed in claims 1 to 3.

8. An anti-inflammatory agent which comprises a diester compound or its pharmacologically acceptable salt as claimed in claims 1 to 3.

9. A method for absorbing ultraviolet radiation or suppressing deposition of melanin pigment which comprises applying to the skin in need thereof a diester compound of maleic acid (or fumaric acid) or its pharmacologically acceptable salt represented by the following formula:

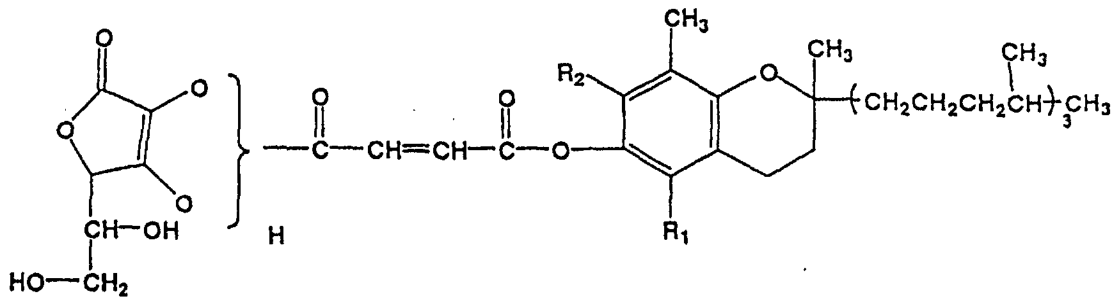

(wherein each of $R_1$ and $R_2$ each is the same as, or different from, the other and designates a hydrogen atom or methyl group).

**10.** A method as claimed in claim 9, wherein the said diester compound or pharmacologically acceptable salt is represented by α-tocopheryl L-ascorbic acid-2-O-maleate (or fumarate.

**11.** A method as claimed in claim 9, wherein the said diester compound or pharmacologically acceptable salt is represented by α-tocopheryl L-ascorbic acid-3-O-maleate (or fumarate).

**12.** A method for suppressing oxidation or scavenging radicals which comprises administering to a subject in need thereof an effective amount of a diester compounds of maleic acid (or fumaric acid) or its pharmacologically acceptable salt represented by the following formula:

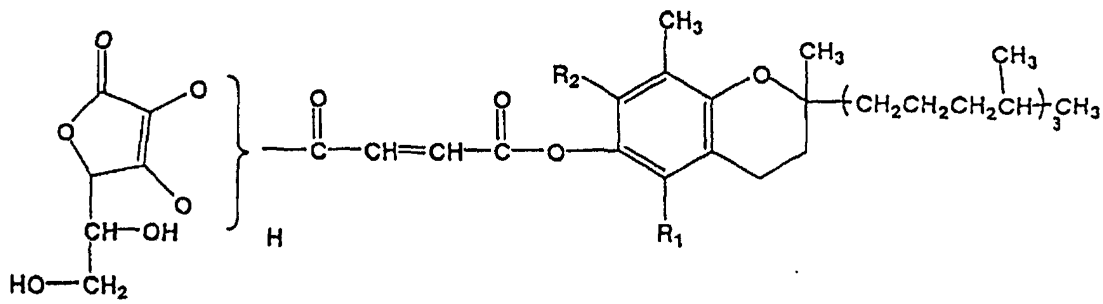

(wherein each of $R_1$ and $R_2$ each is the same as, or different from, the other and designates a hydrogen atom or methyl group).

**13.** A method as claimed in claim 12, wherein the said diester compound or its pharmacologically acceptable salt is represented by α-tocopheryl L-ascorbic acid-2-O-maleate (or fumarate).

**14.** A method as claimed in claim 12, wherein the said diester compound or its pharmacologically acceptable salt is represented by α-tocopheryl L-ascorbic acid-3-O-maleate (or fumarate).

**15.** A method for preventing or treating inflammations which comprises administering to a subject in need thereof an effective amount of a diester compound of maleic acid (or fumaric acid) or its pharmacologically acceptable salt represented by the following formula:

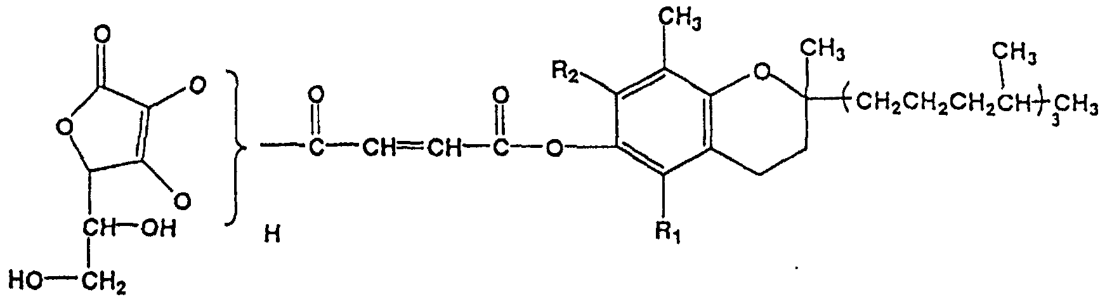

(wherein each of $R_1$ and $R_2$ is the same as, or different from, the other and designates a hydrogen atom or methyl group).

**16.** A method as claimed in claim 15, wherein the said diester compound or its pharmacologically acceptable salt is represented by α-tocopheryl L-ascorbic acid-2-O-maleate (or fumarate).

**17.** A method as claimed in claim 15, wherein the said diester compound or its pharmacologically acceptable salt is represented by α-tocopheryl L-ascorbic acid-3-O-maleate (or fumarate).

**18.** A use in the manufacture of cosmetics of a diester compound of maleic acid (or fumaric acid) or its pharmacologically acceptable salt represented by the following formula:

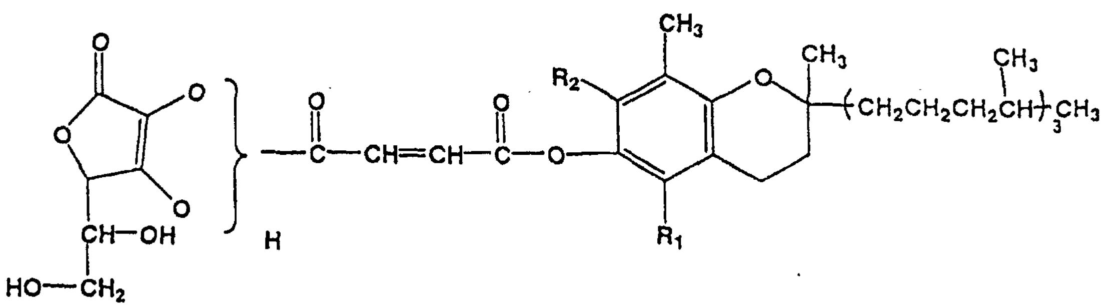

(wherein each of $R_1$ and $R_2$ is the same as, or different from, the other and designates a hydrogen atom or methyl group).

**19.** A use as claimed in claim 18, wherein the said diester compound or its pharmacologically acceptable salt is represented by α-tocopheryl L-ascorbic acid-2-O-maleate (or fumarate).

**20.** A use as claimed in claim 18, wherein the said diester compound or its pharmacologically acceptable salt is represented by α-tocopheryl L-ascorbic acid-3-O-maleate (or fumarate).

**21.** A use in the manufacture of antioxidants or radical scavengers of a diester compound of maleic acid (or fumaric acid) or its pharmacologically acceptable salt represented by the following formula:

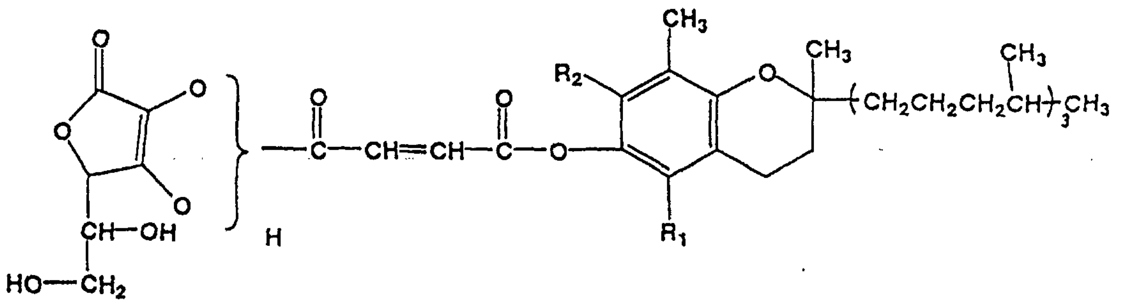

(wherein each of $R_1$ and $R_2$ is the same as, or different from, the other and designates a hydrogen atom or methyl group).

**22.** A use as claimed in claim 21, wherein the diester compound or its pharmacologically acceptable salt is represented by α-tocopheryl L-ascorbic acid-2-O-maleate (or fumarate).

**23.** A use as claimed in claim 21, wherein the said diester compound or its pharmacologically acceptable salt is represented by α-tocopheryl L-ascorbic acid-3-O-maleate (or fumarate).

**24.** A use in the manufacture of pharmaceuticals of a diester compound of maleic acid (or fumaric acid) or its pharmacologically acceptable salt represented by the following formula:

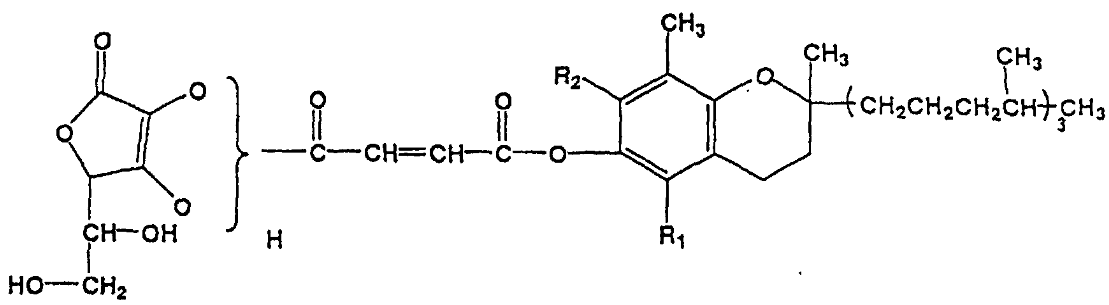

(wherein each of $R_1$ and $R_2$ is the same as, or different from, the other and designates a hydrogen atom or methyl group).

**25.** A use as claimed in claim 24, wherein the said diester compound or its pharmacologically acceptable salt is represented by α-tocopheryl L-ascorbic acid-2-O-maleate (or fumarate).

**26.** A use as claimed in claim 24, wherein the said diester compound or its pharmacologically acceptable salt is represented by α-tocopheryl L-ascorbic acid-3-O-maleate (or fumarate).

**27.** A use in the manufacture of anti-inflammatory agents of a diester compound of maleic acid (or fumaric acid) or its pharmacologically acceptable salt represented by the following formula:

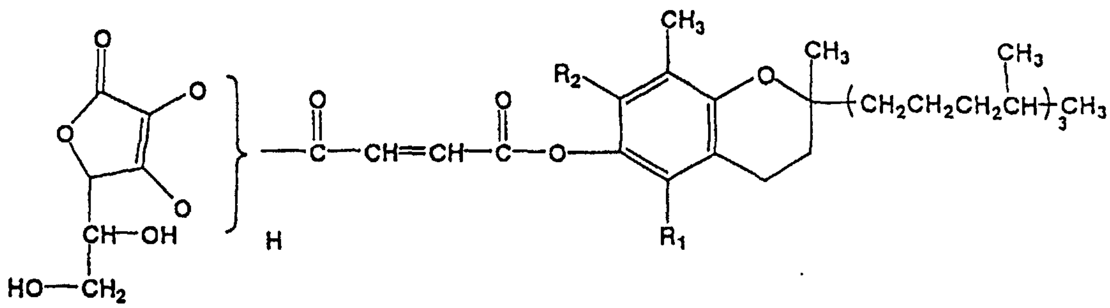

(wherein each of $R_1$ and $R_2$ is the same as, or different from, the other and designates a hydrogen atom or methyl group).

**28.** A use as claimed in claim 27, wherein the said diester compound or its pharmacologically acceptable salt is represented by α-tocopheryl L-ascorbic acid-2-O-maleate (or fumarate).

**29.** A use as claimed in claim 27, wherein the said diester compound or its pharmacologically acceptable salt is represented by α-tocopheryl L-ascorbic acid-3-O-maleate (or fumarate).

FIG. 1

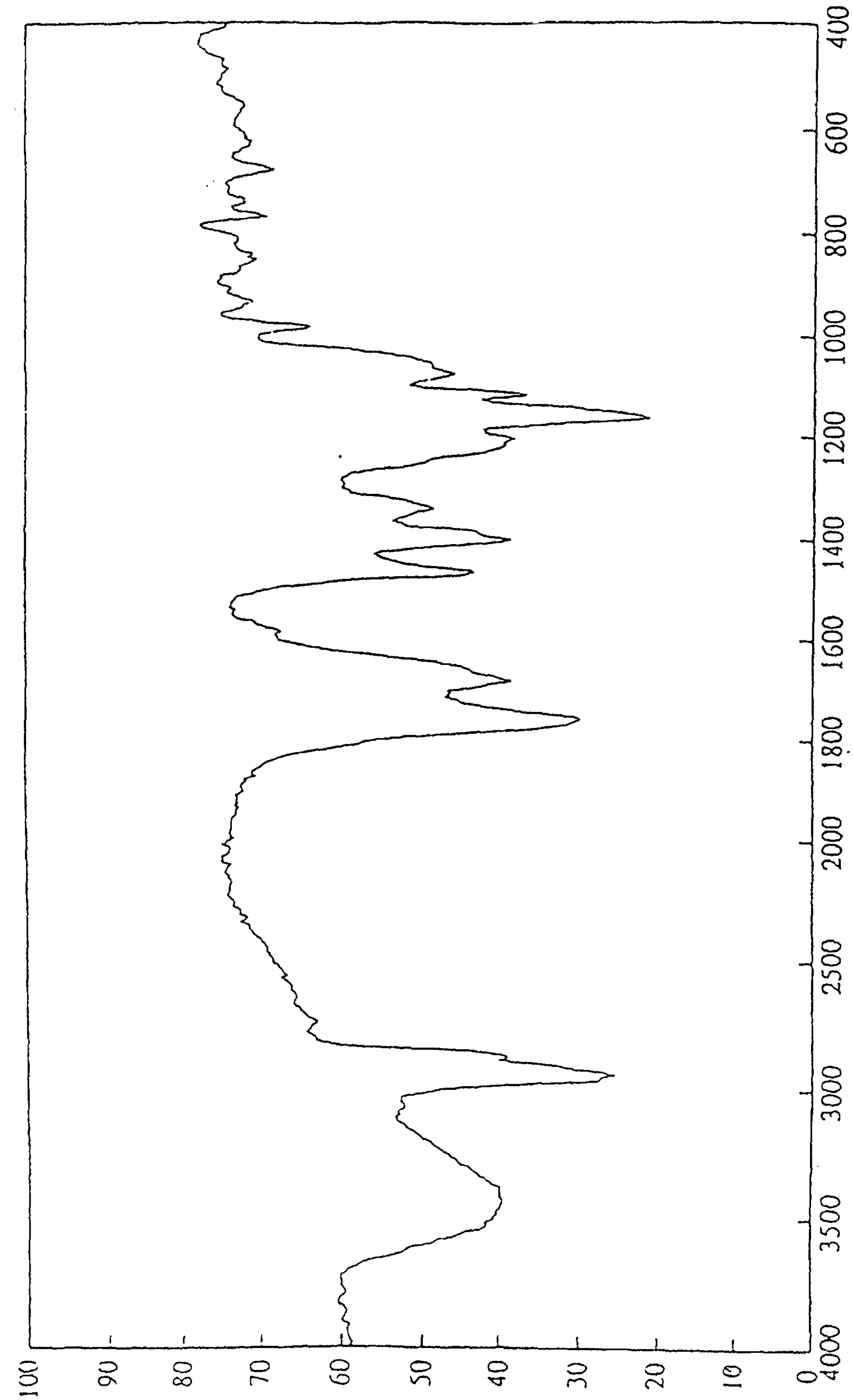
100
90
80
70
60
50
40
30
20
10
0
4000
3500
3000
2500
2000
1800
1600
1400
1200
1000
800
600
400
wave number (cm-1)

## INTERNATIONAL SEARCH REPORT

International application No.
PCT/JP00/04575

**A. CLASSIFICATION OF SUBJECT MATTER**
Int.Cl$^7$ C07D407/12, A61K31/375, 7/00, 7/42, A61P29/00

According to International Patent Classification (IPC) or to both national classification and IPC

**B. FIELDS SEARCHED**

Minimum documentation searched (classification system followed by classification symbols)
Int.Cl$^7$ C07D407/12, A61K31/375, 7/00, 7/42, A61P29/00

Documentation searched other than minimum documentation to the extent that such documents are included in the fields searched

| | | | |
|---|---|---|---|
| Jitsuyo Shinan Koho | 1940-1992 | Toroku Jitsuyo Shinan Koho | 1994-1996 |
| Kokai Jitsuyo Shinan Koho | 1971-1992 | Jitsuyo Shinan Toroku Koho | 1996-1999 |

Electronic data base consulted during the international search (name of data base and, where practicable, search terms used)
CAPLUS(STN),REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP, 4-149113, A (Kanebo, LTD.),<br>22 May, 1992 (22.05.92) (Family: none)<br>Claims; examples 5, 14, 19 | 1-5,7,18-20, 24-26<br>6,8,21-23, 27-29 |
| Y | EP, 768314, A1 (Senju Pharmaceutical Co., Ltd.),<br>16 April, 1997 (16.04.97)<br>& JP, 9-165394, A & US, 5750516, A<br>& CA, 2186654, A | 6,21-23 |
| Y | EP, 236120, A (Senju Pharmaceutical Co., Ltd.),<br>09 September, 1987 (09.09.87)<br>& JP, 62-205091, A & US, 4914197, A<br>& CA, 1269669, A | 8,27-29 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>08 August, 2000 (08.08.00) | Date of mailing of the international search report<br>22 August, 2000 (22.08.00) |
| Name and mailing address of the ISA/<br>Japanese Patent Office<br><br>Facsimile No. | Authorized officer<br><br>Telephone No. |

Form PCT/ISA/210 (second sheet) (July 1992)

**INTERNATIONAL SEARCH REPORT**

International application No.
PCT/JP00/04575

**Box I Observations where certain claims were found unsearchable (Continuation of item 1 of first sheet)**

This international search report has not been established in respect of certain claims under Article 17(2)(a) for the following reasons:

1. ☒ Claims Nos.: 9-17
because they relate to subject matter not required to be searched by this Authority, namely:

Claims 9 to 17 pertain to methods for treatment of the human body by therapy, and thus relate to subject matters which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)